Europäisches Patentamt

European Patent Office

Office européen des brevets

(11) Numéro de publication: **0 269 496 B1**

# FASCICULE DE BREVET EUROPEEN

(45) Date de publication de fascicule du brevet:
**13.11.91**

(21) Numéro de dépôt: **87402467.2**

(22) Date de dépôt: **03.11.87**

(51) Int. Cl.⁵: **G01F 15/06, G06M 3/00, B05B 11/00, A61J 7/00**

(54) **Dispositif de vaporisateur doseur muni d'un moyen de comptage des doses émises.**

(30) Priorité: **06.11.86 FR 8615476**

(43) Date de publication de la demande:
**01.06.88 Bulletin 88/22**

(45) Mention de la délivrance du brevet:
**13.11.91 Bulletin 91/46**

(84) Etats contractants désignés:
**AT BE CH DE ES FR GB GR IT LI LU NL SE**

(56) Documents cités:
**EP-A- 0 114 617**
**DE-A- 2 033 596**
**FR-A- 2 400 693**
**GB-A- 2 037 045**
**US-A- 2 357 940**

(73) Titulaire: **VALOIS Société Anonyme dite:**
**Boîte Postale G Le Prieuré**
**F-27110 Le Neubourg(FR)**

(72) Inventeur: **Liesse, Maurice**
**2 rue Sainte-Colombe**
**F-89100 Saint Denis Les Sens(FR)**

(74) Mandataire: **Pinguet, André**
**Cabinet de Proprieté Industrielle CAPRI 28**
**bis, avenue Mozart**
**F-75016 Paris(FR)**

## Description

La présente invention a pour objet un dispositif, éventuellement adaptable, de vaporisateur doseur muni de moyens de comptage des doses émises.

L'invention s'applique aussi bien aux valves dites doseuses, dont le principe utilise la pression d'un gaz propulseur renfermé dans un flacon avec le produit à pulvériser, qu'aux pompes fonctionnant avec l'énergie développée par l'enfoncement d'un bouton poussoir. Ces appareils comportent une tige de soupape qui fait saillie extérieurement hors de la valve ou pompe, et un poussoir engagé sur cette tige. La tige et la valve (pompe) ont, dans leur ensemble, une structure de révolution autour de l'axe de la tige, que l'on désignera ici aussi par axe de poussée.

Dans les applications médicales en particulier, un utilisateur doit pulvériser (dans les narines, la gorge, etc.) un certain nombre de doses, par exemple quotidiennement. Afin d'éviter des erreurs, et pour simplifier l'application, il a paru intéressant de disposer d'un vaporisateur muni de moyens permettant d'afficher le nombre de doses pulvérisées, ou restant à pulvériser si l'on a affiché préalablement le nombre de doses à pulvériser (compteur ou décompteur).

On a déjà proposé, dans le brevet européen 0 114 617 une valve doseuse qui comporte un compteur de l'actionnement du bouton-poussoir. Le compteur comporte une couronne mobile, munie de surfaces obliques contre lesquelles peuvent venir pousser des surfaces obliques complémentaires solidaires du poussoir. La descente du poussoir provoque une rotation partielle de la couronne. La rotation est complétée par un système à cliquets, compliqué, coûteux à réaliser, et peu sûr. La présente invention a pour objet la réalisation d'un système simple, peu coûteux, et fiable.

Ce résultat est obtenu, conformément à la présente invention par un dispositif de dosage commandé par un poussoir à mouvement axial sans pouvoir tourner sur lui-même, pour débiter une dose d'une substance capable de s'écouler à partir d'un récipient, lors d'une course du poussoir, et avec un dispositif de comptage des courses présentant une couronne de comptage coaxiale au poussoir, pouvant tourner autour de l'axe de poussée sans pouvoir se déplacer axialement lors des courses du poussoir, caractérisé en ce qu'une denture périphérique orientée axialement, et une lame souple inclinée par rapport à l'axe disposée dans le profil de la couronne et en regard de la denture, sont solidaires chacun respectivement de la couronne et du poussoir au inversement, l'extrémité de la lame souple étant à une distance de la couronne inférieure à la longueur de la course du poussoir.

D'autres caractéristiques et avantages de l'invention apparaîtront au cours de la description qui va suivre, donnée à titre d'exemple non limitatif, en regard des dessins ci-joints, et qui fera bien comprendre comment l'invention peut être réalisée.

Sur les dessins :

la figure 1 est un schéma de principe du fonctionnement de l'invention,

la figure 2 est une vue en perspective d'un des deux éléments du système de comptage,

la figure 3 est une perspective d'une des pièces du dispositif, la couronne comportant l'autre élément du système de comptage, et

la figure 4 est une vue en élévation, avec parties en coupe d'un exemple de dispositif selon l'invention.

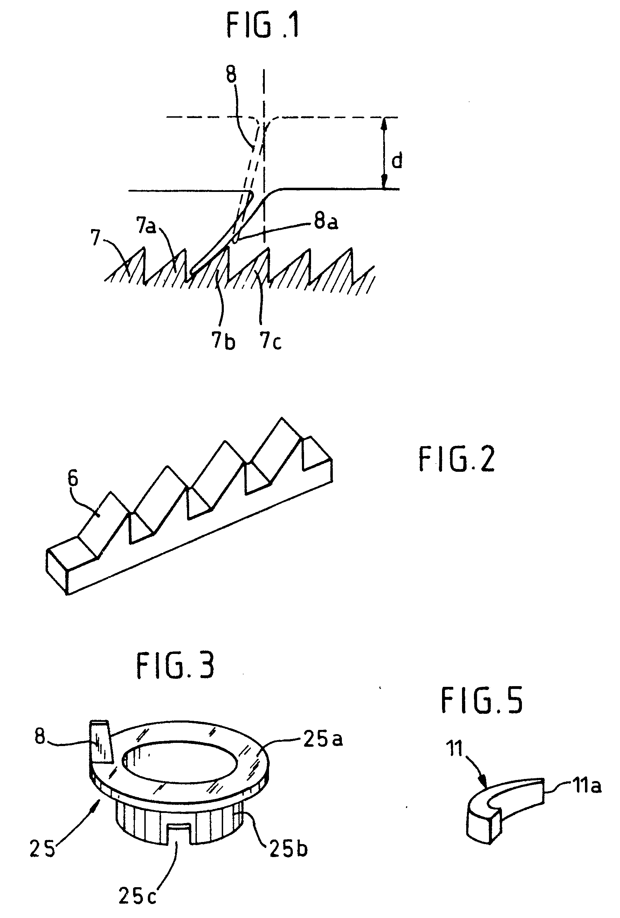

La figure 1 représente un exemple de principe où la totalité d'un mouvement de rotation de la couronne 25 est obtenue pendant le seul mouvement de descente de l'ergot 8. La couronne 25 mobile porte, suivant un disque horizontal, une série de dents 7 telles que représentées sur la figure 2 , et un ergot 8 se déplace circulairement au-dessus de cette série de dents 7. L'ergot 8 est constitué par une lamelle souple 8, et est incliné, dans le sens de rotation voulu pour privilégier le sens de la flexion. En position de repos, le bord inférieur 8a de l'ergot 8 est placé légèrement au-dessus des arêtes supérieures des dents 7. Quand on fait marcher le vaporisateur, en enfonçant le poussoir, l'ergot 8 descend, et sa pointe 8a se loge entre deux dents 7a, 7b ; elle se cale dans le fond, et la continuation de la descente provoque la flexion de l'ergot 8 et la poussée de la dent 7a vers la gauche de la figure. La longueur de la nervure (ergot) 8 peut être choisie suffisante pour que la poussée sur la dent 7a provoque une rotation de la couronne telle, qu'après remontée de l'ergot 8, sa pointe 8a se trouve au-dessus de l'intervalle entre les deux dents suivantes 7b, 7c. La distance D entre le niveau de repos, en trait interrompu, et le niveau bas, en trait plein, représente la course du bouton poussoir pour expulser une dose. On remarquera que les rôles de la crémaillère 7 et de l'ergot 8 sont symétriques : la crémaillère 7 peut être solidaire du poussoir et l'ergot 8 peut être solidaire de la couronne mobile. Avantageusement, la couronne est montée à frottement doux pour éviter une rotation indésirée.

Afin de rendre plus aisé l'emploi du dispositif selon l'invention, il est intéressant de définir une position origine pour la rotation de la couronne, et des moyens pour l'y amener.

Dans le cas de la figure 1, en position de repos, on a noté que l'extrémité inférieure 8a de l'ergot est située au-dessus des arêtes supérieures des dents 7. Donc, dans cette position, la couronne peut tourner librement. L'utilisateur peut donc amener l'index sur un chiffre désiré. Selon le sens de la graduation, il peut amener l'index sur le zéro, et le dispositif avance dans le sens des chiffres croissants, c'est le cas du compteur. Avec une graduation en sens inverse, l'utilisateur amène l'index sur le chiffre désignant le nombre des doses à prendre, et à chaque dose, l'index descend d'un point. L'utilisateur peut voir à chaque instant le nombre des doses qui lui restent à prendre.

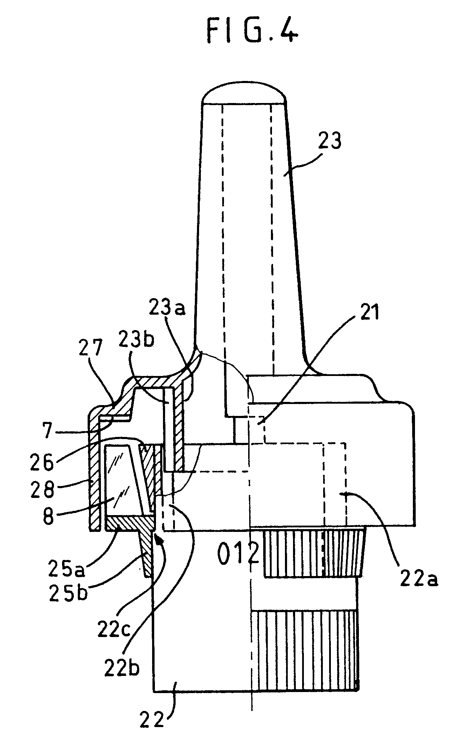

La figure 4 représente en élévation, avec coupe partielle, un mode de réalisation d'un dispositif correspondant au principe décrit en regard de la figure 1. La soupape 21 (valve ou pompe) peut être montée sur un flacon quelconque (non représenté) au moyen d'une bague 22 qui peut être vissée sur le goulot du flacon, ou encliquetée, ou sertie, et maintenue de façon à ne pas pouvoir tourner en usage normal (c'est évidemment très préférable, mais pas indispensable au sens de la présente invention qui prévoit une rotation de la couronne 25 par rapport à la bague 22). Un bouton poussoir 23, de forme adaptée au produit à pulvériser, est emmanché sur la soupape. Pour assurer un bon guidage du poussoir 23 par rapport au flacon, le poussoir comporte une jupe 23a, s'étendant vers le bas, coulissant dans un prolongement tubulaire 22a de la bague 22. Des moyens sont prévus pour empêcher le poussoir 23 de tourner par rapport à la bague 22, par exemple une nervure 23b à l'extérieur de la jupe 23a, coopérant avec une rainure 22b formée dans le prolongement tubulaire 22a. Le poussoir comporte une partie circulaire horizontale 27 garnie d'une crémaillère de dents 7, correspondant à celles de la figure 1 ou de la figure 2, et orientée vers le bas. Le poussoir est enfin complété par une garniture périphérique 28 enveloppant l'ensemble. Une couronne 25 (figure 3) est montée sur un épaulement 22c de la bague 22. Cette couronne 25 comporte une collerette plane 25a et une jupe 25b. La jupe se raccorde à la collerette à une petite distance du bord intérieur de façon à former un épaulement qui coopère avec l'épaulement 22c de la bague 22. La jupe 25b est prévue pour glisser sur la partie supérieure de la bague 22 et comporte une fenêtre 25c qui a pour objet de laisser apparaître un des chiffres imprimés sur le haut de la bague 22, repères de position de la couronne 25 au cours de sa rotation, c'est-à-dire le nombre de doses émises, ou restant à émettre, selon le sens de graduation des chiffres. La collerette 25a porte un ergot 8 en forme de lamelle souple inclinée dans le sens désiré de rotation. Cette lamelle 8 coopère avec la crémaillère de dents 7, comme décrit en regard de la figure 1. Une bague 26 sert à maintenir en position la couronne 25. Elle est emmanchée sur le prolongement tubulaire 22a de la bague 22, le bas du poussoir et la couronne 25 délimitant un espace annulaire 29 à l'intérieur duquel interfèrent l'ergot et des éléments en saillie. Le fonctionnement résulte des explications données en regard de la figure 1. Selon le sens de l'ordre des chiffres, les enfoncements successifs du poussoir provoqueront des rotations qui feront apparaître dans la fenêtre 25c des chiffres croissants ou décroissants selon le choix désiré. Dans le sens décroissant, par exemple, l'utilisateur placera dans la fenêtre 25c le nombre de doses à prendre, et à chaque dose expulsée, le chiffre affiché descendra d'une unité. On peut prévoir deux lamelles 8 diamétralement opposées, ou plus.

**Revendications**

1. Dispositif de dosage commandé par un poussoir (23) à mouvement axial sans pouvoir tourner sur lui-même, pour débiter une dose d'une substance capable de s'écouler à partir d'un récipient, lors d'une course du poussoir (23), et avec une dispositif de comptage des courses, présentant une couronne (25) de comptage coaxiale au poussoir (23), pouvant tourner autour de l'axe de poussée sans pouvoir se déplacer axialement lors des courses du poussoir (23) caractérisé en ce qu'une denture (7) périphérique orientée axialement, et une lame souple (8) inclinée par rapport à l'axe disposée dans le profil de la couronne (25) et en regard de la denture (7), sont solidaires chacun respectivement de la couronne (25) et du poussoir (23) au inversement, l'extrémité de la lame souple (8) étant à une distance de la couronne (25) inférieure à la longueur de la course du poussoir (23).

2. Dispositif selon la revendication 1, caractérisé en ce que la couronne (25) porte la denture (7) et la lame souple (8) est solidaire du poussoir (23).

3. Dispositif selon la revendication 1, caractérisé en ce que la couronne (25) porte la lame souple (8) et la denture (7) est solidaire du poussoir (23).

4. Dispositif selon une des revendications précitées, caractérisé en ce que la couronne (25) comporte une partie en forme de collerette (25a) et une partie cylindrique (25b) raccordée à la collerette (25a) à une petite distance du bord intérieur de celle-ci, ladite partie cylindrique (25b) comportant une fenêtre (25c) et des chiffres sont inscrits sur une valve (22) à la hauteur de ladite fenêtre (25c), de façon qu'un chiffre apparaisse dans la fenêtre (25c), et que la fraction de tour de la couronne (25) provoquée par un enfoncement du bouton poussoir (23) corresponde à l'écartement des chiffres.

## Claims

1. A metering device actuated by a pushbutton (23), said pushbutton being movable axially and being prevented from rotating on itself, said metering device being suitable for discharging a metered quantity of a substance able to flow out of a container during a stroke of the pushbutton (23), and said metering device having a stroke counting device including a counting ring (25) which has the same axis as the pushbutton (23) and which is able to rotate about the thrust axis and is prevented from moving axially during the strokes of the pushbutton (23), characterized in that peripheral, axially oriented teeth (7) and a flexible blade (8), which is inclined relative to the axis and which is disposed in the profile of the ring (25) and facing teeth (7), are each bound respectively to the ring (25) and to the pushbutton (23) or inversely, the end of the flexible blade (8) being at a distance from the ring (258) less than the length of the stroke of the pushbutton (23).

2. A device according to claim 1, characterized in that the ring (25) carries the teeth (7), and the flexible blade (8) is fixed to the pushbutton (23).

3. A device according to claim 1, characterized in that the ring (25) carries the flexible blade (8), and the teeth (7) are fixed to the pushbutton.

4. A device according to any one of the preceding claims, characterized in that the ring (25) includes a flange-like portion (25a) and a cylindrical portion (25b) which is bound to the flange (25a) at a small distance from the inner edge thereof, said cylindrical portion (25b) having a window (25c), and numbers are written on a valve (22) level with said window (25c), such that a number appears in the window (25c) and that the fraction of a turn through which the ring (25) is rotated by pressing the pushbutton (23) corresponds to the pitch of the numbers.

## Patentansprüche

1. Dosiervorrichtung, die von einem Drücker (23), der sich axial bewegen, aber nicht um sich selbst drehen kann, gesteuert wird zur Ausgabe einer Dosis einer Substanz, die bei Bewegung des Drückers (23) aus einem Behälter austreten kann, und mit einer Vorrichtung zum Zählen der Bewegungen, die eine zum Drükker (23) koaxial liegende Zählkrone (25) aufweist, die sich während der Bewegungen des Drückers (23) um die Druckachse drehen kann, ohne sich axial verschieben zu können, dadurch gekennzeichnet, daß eine axial ausgerichtete periphere Zahnung (7) und eine in Bezug auf die Achse geneigte, im Profil der Krone (25) und gegenüber der Zahnung (7) angeordnete biegsame Lamelle (8) je fest mit der Krone (25) bzw. dem Drükker (23) oder umgekehrt verbunden sind, wobei das Ende der biegsamen Lamelle (8) sich in einem Abstand zur Krone (25) befindet, der geringer als die Weglänge des Drückers (23) ist.

2. Vorrichtung nach Anspruch 1, dadurch gekennzeichnet, daß die Krone (25) die Zahnung (7) trägt und die biegsame Lamelle (8) fest mit dem Drücker (23) verbunden ist.

3. Vorrichtung nach Anspruch 1, dadurch gekennzeichnet, daß die Krone (25) die biegsame Lamelle (8) trägt und die Zahnung (7) fest mit dem Drücker (23) verbunden ist.

4. Vorrichtung nach einem der vorhergehenden Ansprüche, dadurch gekennzeichnet, daß die Krone (25) einen Bereich in Form eines Kragens (25a) und einen zylindrischen Bereich (25b) aufweist, der in einem geringen Abstand zum inneren Rand des Kragens an diesem befestigt ist, wobei dieser zylindrische Bereich (25b) ein Fenster (25c) aufweist und Zahlen auf einem Ventil (22) in Höhe dieses Fensters (25c) aufgezeichnet sind, so daß eine Zahl im Fenster (25c) erscheint, und daß der Drehungsbruchteil der Krone (25), der von einem Eindrücken des Drückers (23) hervorgerufen wird, dem Abstand der Zahlen entspricht.

FIG .1

8

d

7    7a    8a

7b    7c

FIG.2

6

FIG.3

8    25a

25    25b

25c

FIG.5

11    11a

# FIG.4